# EUROPEAN PATENT APPLICATION

(11) **EP 3 552 570 A1**
(43) Date of publication of application: **16.10.2019**
(21) Application number: 18167171.0
(22) Date of filing: 13.04.2018
(51) Int. Cl.: A61B 18/20, A61B 18/00

(54) **SKIN OR HAIR TREATMENT DEVICE FOR EMITTING HIGH INTENSE TREATMENT LIGHT**

(71) Applicant: Braun GmbH, 61476 Kronberg im Taunus (DE)
(72) Inventor: Beerwerth, Frank, 61476 Kronberg (DE); Harttmann, Brigitte, 61476 Kronberg (DE); Mandre, Shyam Kishan, 61476 Kronberg (DE); Neyer, Christian, 61476 Kronberg (DE); Schmitt, Timo, 61476 Kronberg (DE); Kruch, Torben, 61476 Kronberg (DE); Bogatirsky, Dmitri, 65760 Eschborn (DE)
(74) Representative: P&G Patent Germany

(57) **Abstract**

A skin or hair treatment device for emitting intense light radiation (113) with an integrated sensor is describe comprising a housing (101), a treatment light source (110, 210) disposed inside the housing (101) of the treatment device (100) for illuminating through a device treatment window (102) a surface (1), in particular a skin surface, disposed outside the housing (101) in front of the device treatment window (102), a sensor system (120, 220, 320) disposed inside the housing (101) of the treatment device (100). The sensor system (120, 220, 320) comprises a sensing light source (121, 221, 321) for illuminating the surface (1) disposed outside the housing (101) in front of the device treatment window (102) with sensing light, a light sensor (123, 223, 323) for detecting the sensing light on said illuminated surface (1), the light sensor (123, 223, 323) being directed towards the illuminated surface (1) in front of the device treatment window (102) and a control circuit having a processor, the control circuit being adapted to control the treatment light source (110, 210), the sensing light source (121, 221, 321), and the light sensor (123, 223, 323). Controlling of the sensing light source (121, 221, 312) comprises switching off and on a sensing light, controlling of the treatment light source (110) comprises full intensity control of the high intense treatment light (113), and controlling the light sensor (123, 223, 323) comprises collecting data from the light sensor (123, 223, 323) and evaluating the data. The light sensor (123, 223, 323) is an image sensor, and the sensor system (120, 220, 320) and the control circuit are adapted to be used for at least two, preferably all three of the following measurements of the skin color, of the distance between the treatment device (100) and the surface (1), and of a movement or velocity of the treatment device (100) relative to the surface (1).

## Description

### FIELD OF THE INVENTION

Skin or hair treatment device for emitting high intense treatment light with an integrated sensor comprising a sensor light source (preferably a light source such as a laser diode or light emitting diode) and a light detector.

The skin or hair treatment device may be a hair management device, in particular an intense pulsed light (IPL) hair management device such as a hair removal device. By irradiating the intense light onto the skin surface and the hair, the intense light targets to the pigment melanin in the hair follicle helping to put the hair to sleep and to stop or reduce growth of the hair. The light is absorbed by the melanin and thereby converted to heat. The local heat development leads to obliteration of the root of the hair. The skin or hair treatment or hair removal device is a household appliance for a use by a private user.

### BACKGROUND OF THE INVENTION

The EP 1 771 121 B1 describes a device for reducing hair growth on a subject. The device comprises a housing having an opening to allow radiation to pass there through, a treatment light source disposed within the housing, a sensor light source disposed within the housing, and an optical sensor. The said optical sensor is arranged for detecting the reflection and scattering properties of the subject at the treatment location from a reflected and scattered sensor beam emitted by the sensor light source. To this aim, the optical sensor is conductively linked to the sensor light source and the treatment light source such that the treatment light source and the sensor light source share at least a portion of an optical path disposed within the housing. The optical sensor might comprise a mechanical or optical movement sensor and a sensor for skin recognition based on optical properties of the skin (reflection and scattering). The optical arrangement defining the optical path for the treatment light and the sensor light require same space leading to quite large devices, many optical components and a complex mechanical design, which finally gets bulky. This leads to high production costs.

### SUMMARY OF THE INVENTION

It is thus an object of the present invention to provide a sensor system for the treatment device able to perform more measurements with less detection means in a compact arrangement and to allow a more precise control of the treatment device.

This object is solved by a skin or hair treatment device for emitting high intense treatment light with an integrated sensor comprising a housing; a treatment light source disposed inside the housing of the treatment device for illuminating through a device treatment window a surface, in particular a skin surface, disposed outside the housing in front of the device treatment window; a sensor system disposed inside the housing of the treatment device, said sensor system comprising a sensing light source for illuminating the surface disposed outside the housing in front of the device treatment window with sensing light and a light sensor for detecting the sensing light on said illuminated surface, the light sensor being directed towards the illuminated surface in front of the device treatment window; and a control circuit having a processor, the control circuit being adapted to control the treatment light source, the sensing light source, and the light sensor, wherein controlling of the sensing light source comprises switching off and on a sensing light (optionally also including an intensity control of the sensing light), wherein controlling of the treatment light source comprises full intensity control of the high intense treatment light (including switching off and on), and wherein controlling the light sensor comprises collecting data from the light sensor and evaluating the data. The light sensor is an image sensor (i.e. an image light sensor, such as a digital image sensor), and the sensor system and the control circuit are adapted to be used for at least two, preferably all three of the following measurements of the skin color (skin color measurement), of the distance between the treatment device and the surface (distance measurement) and/or of a movement or velocity of the treatment device relative to the surface (movement detection or velocity measurement).

For all these measurements of the sensor system, the identical (only one) sensing light source and the identical (only one) image sensor can be used. The sensor system might be mechanically connected to the treatment light source. According to one embodiment, the treatment light source emitting the high intense light for skin treatment is a multi-chip LED module. For this embodiment, the sensor system is an integral part of the LED module or integrated into a cut-out of the LED module. The proposed skin or hair treatment device thus has a compact sensor system that can be integrated into a module of the treatment light source and allows a very compact device with multiple sensor functions performed by one single sensor system.

### BRIEF DESCRIPTION OF THE DRAWINGS

- Figure 1: shows a cut through an embodiment of the skin or hair treatment device according to the proposal;
- Figure 2: shows a top view on the treatment light source (as seen through a device treatment window of the embodiment shown in Figure 1);
- Figure 3: shows a top view on the planar structure of the treatment light source of another embodiment according to the proposal, the planar structure also carrying the sensor system;
- Figure 4: shows a cut through the sensor system according to Figure 3;
- Figure 5: shows a cut through the sensor system of an alternative example according to the proposal;
- Figure 6: shows a schematic view of the sensor system according Figure 5; and
- Figure 7: shows an example structure of the optical arrangement according to Figure 4.

### DETAILED DESCRIPTION OF THE INVENTION

Before describing advantageous embodiments of the invention related to the Figures, different aspects of the invention are described more in detail. These aspects disclose further features, advantages and possibilities of use of the present invention that might be combined in any useful combination. All features described and/or shown in the drawings are subject matter of the invention, irrespective of the grouping of the features in the claims and/or their back references.

The skin or hair treatment device for emitting high intense treatment light has in line with the invention a sensor system that is integrated into a module of the treatment light source. It does not require extra space in the treatment device and is thus very compact, allowing nevertheless multiple measurements using the one image light sensor. The measurements performed might be used to control the treatment light source for adapting the intensity of the high intense treatment light and/or for realizing safety features of the treatment device.

The sensing light source is used by the sensor system for testing the surface and the treatment light source is used for emitting intense light radiation for skin treatment. The sensor system is used for performing measurements testing the surface, in particular skin including the surface and tissue of the skin as the light penetrates to some extent into the skin disposed outside the housing in front of the device treatment window. A typical value for the penetration into the skin might be about 2 to 5 mm. The measurements for testing the surface are performed by the processor of the control circuit, and might comprise testing for the presence of a surface and for physical properties of the surface, such as the color of the skin defined by the melanin content below the skin surface. The measurement of the physical properties of the skin might also be used for an indication that the detected surface is a skin surface.

It is thus clear to the one skilled in the art that the surface, preferably skin surface, is not part of the proposed skin treatment device but used as a functional feature to describe the function of the light source as a light emitting device and the sensor system as a light detecting device. The sensor system and the control circuit are adapted to test the space in front of the device treatment window, and to determine (1) whether a surface can be detected in front of the device treatment window and (2) whether a detected surface is a skin surface. Accordingly, the terms "surface" and "skin surface" might be understood as equivalents in this text.

The light emitted is intended to illuminate a skin area and the light detecting image sensor is intended the detect light back scattered (reemitted) and/or reflected from the illuminated area. Accordingly, the light sources and the sensor system are disposed inside the housing such that sensing light emitted from the sensing light source is passing through an opening in the device (i.e. the device housing) to the surface (skin surface) and that light reflected and/or back scattered through the device treatment window into the housing in a certain direction is captured by the light sensor. This might be performed by a suited optical arrangement of the sensor element in front of the light detecting sensor. The emitted sensing light might leave the housing through the device treatment window as opening in the device or any other window disposed in a suited arrangement in the housing.

Preferably, at least the measurement regarding the skin color and the distance between the treatment device and the skin surface are performed by the sensor system. The sensor system and the control circuit are adapted to perform the distance measurement between the treatment device and the skin by determining the distance between the device treatment window of device and a surface being assumed to be the skin surface. For this measurement, a light pattern is projected to the surface by the light sensing source, and the projected light pattern is imaged on the surface with the image sensor. The distance of the surface is calculated based on triangulation and a displacement of the projected light pattern or a distance of the projected light pattern to an identified position in the image. The sensor system is further adapted to perform the skin color measurement by illuminating the surface with light from the sensing light source or the treatment light source, and by detecting the light scattered back from the surface being assumed to be the skin surface.

It is possible to perform both measurements within one measuring step by evaluating the measurement signal obtained in one measuring step with two different evaluation procedures. In one embodiment, the same image of the light emitted from the sensing light source might be used.

A proposed wavelength selected for the sensing light source may for example be in the visible light range of 440 nm to 680 nm, especially blue/green light 440 nm to 550 nm, esp. blue 440 nm to 480 nm or green 500 nm to 550 nm. Visible light, in particular blue and/or green light, has the advantage that the absorption of melanin in skin is increasing with lower wavelengths. The skin structure due to melanin is therefore better visible with lower wavelength leading to higher reliability of the movement detection or velocity measurement. Further, the difference of light remission for light skin and dark skin is higher also due to higher absorption of the melanin at lower wavelengths. This reduces measuring tolerances and increases the safety margin of the device for skin color determination.

As already described, a proposed implementation of the distance measurement between the device treatment window and the skin surface is based on a projection of a light pattern, e.g. a dot, a line or any other recognizable pattern, such as a special geometrical form, through a suited opening in the device creating visible light marks on the skin disposed in front of the device treatment window.

The distance measurement is based on triangulation via the light marks on skin, said light marks moving on the skin with changing the distance between the device treatment window and the (skin) surface. Accordingly, the position of the light mark on the skin in the image taken by the image light sensor is a measure for this distance. Movement of the light mark (compared to a surface disposed in front of the device treatment window) might accordingly be detected by comparing the position of the light mark in the image to a predefined position of the light mark when the surface is disposed in front of the device treatment window.

Generation of the pattern can be done via imaging of the light output of a single diode onto the window area via lenses (diffractive or refractive optical elements). Another proposed way is the generation of the light pattern via diffractive lenses located in front of the sensing light source. Further, the projected light pattern might be generated via a landmark on the output window of the device opening (e.g. the device treatment window) and illumination with a point source off-axis. The shadow of the landmark (as a pattern projection) will move in the image depending on the skin distance from the window.

According to a further proposed embodiment (in any combination with features described before or thereinafter), the sensing system can be made to use a linearly or circularly polarized light source emitting linearly or circularly polarized light in combination with a polarization sensitive light sensor. A polarization sensitive light sensor may be obtained by a combination of the image (light) sensor and a polarization filter disposed in front of the sensor. The advantage is an improved measurement of the skin color/pigmentation level. Using linearly polarized light, the light back scattered from the skin surface, which does not contain any skin color information, can be eliminated via crossed polarizers (polarizer of the sensor is 90° rotated vs. the polarization of the light source). The skin color/pigmentation level measurement becomes less sensitive from distance between skin and window.

A proposed wavelength selected for the treatment light source may for example be in the infrared light range of 700 nm to 1100nm. According to another embodiment, the treatment light source may emit light in the visible range. In this case, the treatment light source, or certain elements of an areal treatment light source comprising an areal distribution of light emitting elements (such as an LED array), may be used also as a sensing light source, in particular for the skin color measurement or a movement detection/velocity measurement.

According to an embodiment of the proposal, the treatment light source of the sensor system may comprise an array of a plurality of light emitting elements arranged in a coplanar arrangement on a planar substrate, such as e.g. a printed circuit board or ceramic base). The plurality of the light emitting elements may be arranged in a matrix array, and may optionally be separately tunable in power (including switchable off and on), either individually or in groups. This might be used for controlling the light intensity as a whole or locally distributed within the device treatment window. According to a proposed embodiment, the light emitting elements may be light emitting diodes (LED) or laser diodes. The light source might be adapted to emit coherent light.

The planar substrate for the treatment light source may be separate from a device printed circuit board with other electronic elements, such as the processor. However, all light emitting elements might be disposed on this one single planar substrate as one lighting module.

In line with the proposal, the light sensor and/or the sensing light source are disposed on said same (only one) planar substrate as the treatment light source. The sensor system, the treatment light sources and the control circuit may be disposed (together with the other electric components of the device integrated into the circuitry or as a separated module) on one common device printed circuit board or another planar substrate.

According to another embodiment of the proposal, the light sensor and/or the sensing light source are disposed in a breakthrough of the planar substrate of the treatment light source. According to one possible embodiment, the light sensor might be arranged on a separate substrate in the breakthrough of the planar substrate of the treatment light source. The separate substrate with the light sensor may be disposed in the same distance to the device treatment window as the planar substrate of the treatment light source. However, it may be preferred that the separate substrate with the light sensor is disposed in a larger distance to the device treatment window than the planar substrate of the treatment light source. In this case, the light sensor is shielded against stray light or diffused light from the treatment light source.

The sensing light source of the sensor element may comprise one light emitting element or more light emitting elements. For example, a proposed sensor system might comprise a sensing light source comprising two light emitting elements and one image light sensor disposed in a linear arrangement with the light emitting elements of the sensing light source disposed on opposite sides of the image light sensor.

According to a proposed embodiment, the (image) light sensor is surrounded by light emitting elements of the treatment light source. For example, the (image) light sensor (or the entire sensor system including the sensing light source) might be disposed within the array of the plurality of light emitting elements of the treatment light source on the same planar substrate as the treatment light source or in the breakthrough of the planar substrate of the treatment light source.

The advantage of any the arrangements described before is a very compact arrangement that might be produced in one direct printed board assembly process. Further, the tested skin surface is disposed directly within the area of the skin surface illuminated with the treatment light. This ensures that the skin surface is tested by the sensor system in a zone actually treated.

In line with the proposal, the sensor system might comprise an optical arrangement disposed in front of the light sensor. The optical arrangement can comprise a lens for focusing an image of the surface or skin surface on the light sensor of the sensor system, e.g. an image sensor such as a digital camera type image sensor as a CCD camera chip or any type of pixeled image sensor. The lens focus might be adapted such that a sharp image of the surface or skin surface is displayed on the sensor if the distance between the device and the surface or skin surface is in a distance intended for use of the skin or hair treatment device.

In such an arrangement, the distribution of light on the surface can be determined for control functions of the device. It allows distance measurements via triangulation and movement measurements via displacement of skin landmarks or light marks.

By using more than one image light sensor embedded within the treatment light source array (e.g. two image light sensors, different areas of the surface, in particular the skin surface, can be examined separately and at the same time. This leads to higher confidence levels of the examination and thus enhances safety of the device.

The proposed optical arrangement might further comprise an interference filter, an absorption filter, one or more blinds and/or one or more lenses.

The interference filter can be used to reduce heating of the light sensor and sensor optics located adjacent to high power light sources such as lasers, flashlights or LED's by radiation. The interference filter is in particular meaningful for narrow band light sources, such as lasers or LED's. It is proposed according to one embodiment to dispose the interference filter adjacent to light output window, i.e. as first element of the optical arrangement passed by light radiation falling into the device through the light output window onto the sensor.

The absorption filter can be used to reduce the intensity of any light disturbing the measurement of the light sensor. Accordingly, the absorption filter may be designed to selectively absorb light in certain frequency ranges not used for the measurements, especially covering the spectral range of the adjacent high intensity treatment light source. The absorption filter may be disposed between the interference filter and the lens. It is proposed to dispose the lens directly adjacent to the light sensor, e.g. with a blind element, such as a pinhole blind or slit blind disposed between the lens and the sensor.

The blind may be designed by one or more of blind elements. One blind element may be a tubular blind surrounding the light sensor in the axial direction (i.e. the direction of the optical axis of the image light sensor). This eliminates stray light of the light source entering the sensor element from the side. Further, one or more blind elements such as pinhole blinds or slit blinds may be disposed ahead of and/or behind each of the filters or (irrespective of any filters) along the optical axis of the sensor element. These blind elements absorb light scattered into the sensor element and disturbing the image of the surface on the sensor.

Summarizing, the interference filter and the absorption filter are blocking treatment light to avoid blinding the light sensor. The blinds are part of the system to remove undesired scattered light.

The optical arrangement may be an injection molded plastic part including all or part of the optical components described before. Injection molding might be performed in one or more molding steps.

In particular, the lens and/or the blind elements may also be injection molded with an optically transparent mold (for the lens) and an optically absorbing mold (for the blind).

Each of the filters or both filters in combination reduce the intensity of disturbing light to a value that does not interfere with the function of the sensor element. The blind helps to reduce any stray light in the optical system. The injection molded part is one single part with the optical path automatically adjusted. It can be disposed on the planar substrate, such as a printed circuit board or ceramic base plate, in a defined distance and position above the sensor (image sensor) using e.g. position elements injection molded at the optical arrangement. Accordingly, the optical arrangement may be assembled by standard circuit board assembling techniques, such as SMD, COB, molding or gluing.

The sensing light source might comprise at least one optical element for projecting a light pattern as a distinctive feature through an opening in the device housing, e.g. the device treatment window, for displaying the light pattern on the surface disposed outside the housing in front of the device treatment window. The optical element is adapted such that position of the light pattern on the surface disposed outside the housing in front of the device treatment window is visible by the image light sensor. In line with the proposal, the control circuit can be adapted to determine the distance between the treatment device and the skin by determining the position and/or movement of the projected light pattern in the image of the surface. To this aim, the axis of projection might be inclined to the optical axis of the image light sensor and its optical arrangement. The (smaller) angle between the projection axis and the optical axis may be between 10° and 80°, and more preferably between 20° and 70° and most preferably between 30° and 55°.

By way of the known inclination of the projection axis relative to the optical axis, the position of the projected light pattern in the image depends on the distance between the device treatment window (or light sensor or treatment device, respectively) and the surface. The distance can be determined by known triangulation methods, for example. In order to mark a certain distance, image areas might be defined that indicate a valid distance between the device treatment window (or light sensor or treatment device, respectively) and the (skin) surface if the projected light pattern is visible in the defined image area. This can be tested by the processor of the control circuit by way of adapted image recognition software implemented on the processor. A specific optical element for projecting the projected light pattern may be adjusted such that the projected light pattern is placed outside the sensor's field of view when device is at an initial position (in which there is a gap between the device treatment window and the surface or skin surface). The image of the distinctive features moves into the field of view of the sensor and/or the defined image area if the gap is closed and the certain distance between the surface and the device treatment window is reached.

The control circuit may be adapted to allow switching on the high intense treatment light depending on the location of the projected light pattern in the image. Further, the control circuit may be adapted to switch the high intense treatment light when the projected light pattern is moving out of a predefined image area. This feature can be part of a safety measure to prohibit that high intense treatment light is directed into the user's eyes.

The control circuit, in particular the processor of the control circuit, may be adapted to control the light intensity dependent on the determined light intensity in light sensor segments indicating the amount of coverage of the device treatment window by the surface, in particular skin surface.

In a specific embodiment, there may be provided at least two levels of light intensity. The first level is contributed to full coverage of the light window by the surface (e.g. an overall intensity level of more than 90% of a known detectable intensity) and provides full light intensity. A second level might be provided contributed to a not full coverage of the light window by the surface (e.g. less than 90% of a known detectable intensity) and provides reduced light intensity.

For a measurement of the skin color (based on a measurement of a melanin density in the skin), the sensing light source might comprise at least one optical element for projecting a light through an opening in the device housing, e.g. the device treatment window, for displaying the light spot on the skin surface. The optical element of the sensing light source used for the skin color measurement might be the same as the optical element of the sensing light source used for the distance measurement. Accordingly, the same sensing light source with the same optical element can be used for both, the distance measurement and the skin color measurement. The control circuit of the treatment device is then adapted to select an image area of the skin surface and to determine a signal related to the luminance value of the image indicative of the melanin content of skin. The luminance value might be given by L* for L*ab or Y for YUV systems known by the one skilled in the art for such measurements. Thus, the image senor is used as a color sensor determining a signal indicative of the skin color which is dependent on the melanin content of the skin.

Known color sensor measurements make use of the optical properties of the skin. If the skin is illuminated with light (i.e. the projected light spot) in a direction inclined with respect to the skin normal, preferably at an angle of about 40° (meaning a range of 20° to 70° or more preferred meaning a range of 30° to 55°), the light is either reflected at the skin's surface disposed in front of the device treatment window of the treatment device, absorbed in the skin, or scattered within the skin and reemitted (back scattered). The light which is scattered in the skin in such a way that it is directed perpendicular to the skin can be detected by the image light sensor. As the light sensor proposed according to the invention is an image sensor, by means of standard image processing software it is possible to select an image area of the light sensor adjacent to the light spot. In this image area, the back scattered light can be reliably measured. It is preferred that the optical axis from the image light sensor to the image area on the surface or skin surface is chosen by the control circuit such that the optical axis is perpendicular (or normal) to the skin surface. This might be achieved by choosing specific light elements and/or placing the one or more optical elements for projecting the light spot in such a geometrical relation that the light spot on the skin surface is positioned in the preferred way according to the proposal. However, this aspect of the invention might also be realized with other arrangements.

As the projection of the light spot occurs preferably along an axis inclined with respect to the device treatment window or skin surface, respectively, stray light can be prevented from being detected easily by the optical arrangement disposed in front of the light sensor, including in particular blinds, apertures, optical filters and/or absorbing materials. Optical (band pass fliter) and/or digital filters may also be used select a suited center wave length of the detected light (such as a wavelength spectrum of about 555nm and a full width half maximum of about 100 nm (corresponding to the Y-sensitivity curve of the norm CIE explained in the following).

The brightness of the color L* corresponds to the Y-signal as defined by the Commission Internationale de L'Eclariage, CIE. This Y-signal might be determined and used by the processor of the correspondingly adapted control circuit to determine the skin color. In line with the proposal, the light source can be automatically switched off if the skin is too dark for the treatment.

For the measurement of the skin color, the sensing light source (or selected light elements of the sensing light source) may be operated in a pulse-mode to detect ambient light (i.e. light detected when the sensing light source or the selected light elements of the sensing light source are switched off). The ambient light signal can be subtracted from the total light signal (with the light source or light elements being switched on) for a signal correction.

For performing a velocity measurement or movement detection, the control circuit is adapted to determine the velocity of the treatment device relative to the surface or skin surface by taking images of a surface disposed in front of the device treatment window using the (image) light sensor with a predetermined frequency, preferably a frequency above 200 Hz, most preferably above 1000Hz, and by identifying at least one visible structure of the skin in the image, locating the identified structure in the consecutively taken image and determining the amount of displacement of the identified structure in the consecutive images. This can be performed with known image processing tools. The basic technology of this technique is known to the one skilled in the art.

With the known frequency of taking the images and the amount of displacement, the velocity of the treatment device relative to any structured surface, in particular a skin surface, can be determined. The control circuit might be arranged to switch off and/or disable switching on the high intense treatment light.

The velocity of the treatment device relative to the surface can thus be determined, or a respective movement of the device can be detected, with a precise optical measurement without the need of any moving parts in the device.

With the proposal a sensor system is proposed that can perform in contrast to the prior art measurements of the skin color, of the distance between the treatment device and the surface and of a movement or velocity of the treatment device relative to the surface with the identical light sensor being an image sensor (i.e. an image light sensor) and the identical sensing light source. Further, any optical arrangement in front of the light sensor and any optical element of the sensing light source can be used in the same form for all these measurements.

A further advantage is that the sensor system (with the light sensor and the sensing light source) can be disposed such that the light emission and detection of the sensor system can make use of the one device treatment window. No other openings in the treatment device are necessary for any signal input or output to be detected or irradiated on the surface. The device treatment window is thus also a flat sensor surface that can be easily cleaned and closes the device tightly (no entrance of fluids or particles). Further, the sensed area of the surface is the same area that is treated by the treatment device.

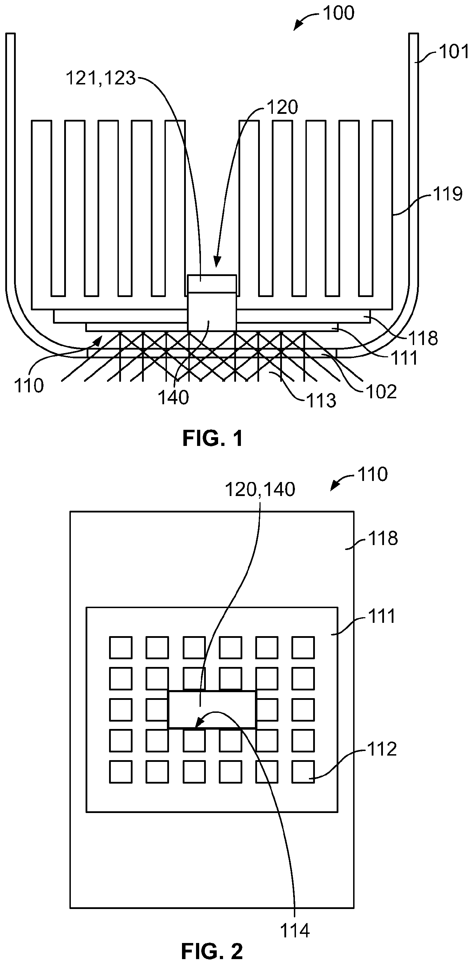

Figure 1 shows a proposed embodiment of a skin or hair treatment device 100 for emitting intense light radiation 113 with an integrated sensor comprising a housing 101. A treatment light source 110 is disposed inside the housing 101 of the treatment device 100 for illuminating through a device treatment window 102 in the housing 101 a surface (not shown in Figure 1). The surface, in particular a skin surface, may be disposed outside the housing 101 in front of the device treatment window 102.

A sensor system 120 is disposed inside the housing 101 of the treatment device 100, said sensor system 120 comprising a sensing light source 121 (not shown in detail in Figure 1) for illuminating the surface disposed outside the housing 101 in front of the device treatment window 102 with sensing light and a light sensor 123 (not shown in detail in Figure 1) for detecting the sensing light on said illuminated surface, the light sensor 123 being directed towards the illuminated surface in front of the device treatment window 102. In front of the light sensor 123 and/or the sensing light source 121 may be placed an optical arrangement 140.

The treatment light source 110 comprises a planar substrate 111, such as a printed circuit board, with light emitting elements (not shown in Figure 1), such as a plurality light emitting diodes LED. The plurality of the light emitting elements may be arranged in a matrix array. The light emitting elements emit the intense light radiation 113, as indicated by the lines in Figure 1 originating at one of the light emitting elements each.

At the rear side of the planar substrate 111 (directed away from the device treatment window 102), there is provided a heat spreader 118 in thermal contact with both, the planar substrate 111 and a heat sink 119 disposed inside the housing 101 and used to spread the heat creating locally at the light emitting elements within the housing 101 of the treatment device 100.

The treatment device 100 further has a control circuit (not shown in the Figures) with a processor, the control circuit being adapted to control the treatment light source 110 as well as the sensing light source 121 and the light sensor 123 of the sensor system 120, wherein controlling of the sensing light source 121 at least comprises switching off and on a sensing light, wherein controlling of the treatment light source 110 possibly comprises full intensity control of the high intense treatment light, and wherein controlling the light sensor 123 comprises collecting data from the light sensor 123 and evaluating the data. The light sensor 123 is an image sensor, such as a CCD-camera sensor, and the sensor system 120 and the control circuit are adapted to be used for at least two, preferably all three, of the measurements of skin (or surface) color, of the distance between the treatment device 100 and the surface (assumed to be skin with or without hair), and/or of a movement or velocity of the treatment device 100 relative to the surface.

Figure 2 shows a view on the planar structure 111 of the light treatment source 110 of the treatment device 100 (seen in the direction of the device treatment window 102). Disposed on the heat spreader 118, and in thermal contact therewith, the planar structure 111 is provided in form of the printed circuit board, typically made of good heat conducting materials, e.g. AlN ceramics, carrying LED-chips as light emitting elements 112. The light emitting elements 112 are arranged in a matrix array. Within the matrix array of the light emitting elements 112 (in the shown example in the center thereof as proposed arrangement), there is provided a breakthrough 114 in the planar structure 111, the heat spreader 118 and the heat sink 119 of the treatment light source 110. The sensor system 120 with its optical arrangement 140 is disposed in said breakthrough 114.

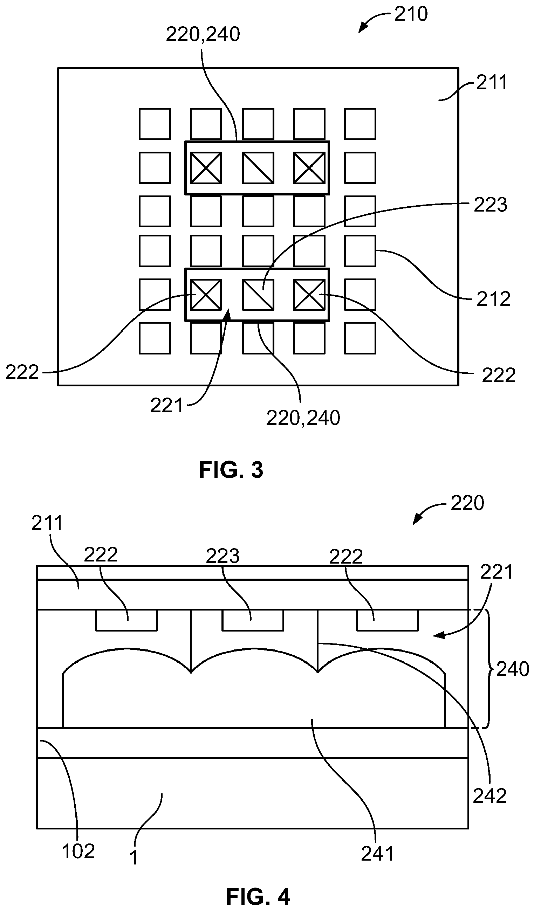

In Figure 3, a view of a planar structure 211 of the light treatment source 210 according to another embodiment is shown. As described with respect to the embedment according to Figure 2, the planar structure 211 also carries LEDs as light emitting elements 222 in a matrix array. However, instead of providing a breakthrough 114 in the planar structure 211, the planar structure 211 also carries the sensor system 220 with its optical arrangement 240 in front of the sensing light source 221 and the light sensor 223. The sensing light source 221 comprises, similar to the treatment light source 110 shown in Figure 2, a plurality of single light emitting elements 222. In the example proposed, the light emitting elements 222 of the sensing light source 221 and the light emitting elements 212 of the treatment light source 210 have about the same size. Further, the light sensor 223 and the light emitting elements 212 of the treatment light source 201 have about the same size, which is denominated also as matrix element size. As the matrix element size of all these elements is to be about equal, the light emitting elements 222 of the sensing light source 221 and the light sensor 223 can be integrated in line with the proposal in the matrix structure of the light emitting elements 212 of the planar structure 211.

Figure 3 shows two sensor systems 220 (with the optical arrangement 240) integrated into the planar structure 211. Each sensor system 220 comprises two light emitting elements 222 and one light sensor 223 disposed in one single line, with the light sensor 223 positioned in the middle between the two light emitting elements 222.

Figure 4 is a cut through the sensor system 220 disposed on the planar structure 211 as shown in Figure 3 with the light sensor 223 disposed in one line between two light emitting elements 222 of the sensing light source 221. Between the device treatment window 102 (which is the same in all embodiments shown in the Figures) and the sensing light source 221 is disposed the optical arrangement 240. The optical arrangement 240 comprises according to a one example a lens 241 having lens elements for each of the light emitting elements 222 of the sensing light source 221 and the light sensor 223. Further, a blind 242 is provided surrounding the sensing light sensor 223 as a tubular blind. This tubular blind of the blind 242 forms kind of an aperture for shielding stray light from entering the light sensor 223.

Outside the housing 101 of the treatment device 100 and shown in direct contact with the device treatment window 104, Figure 4 shows schematically a skin surface 1.

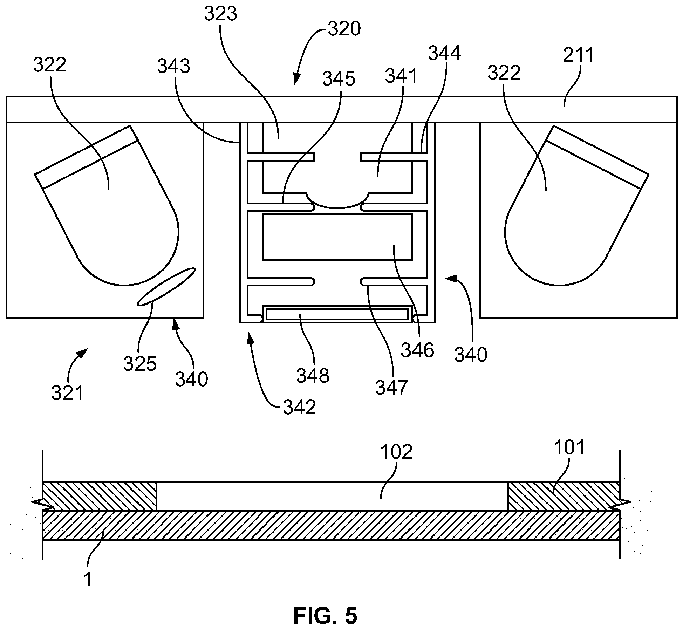

Figure 5 shows a cut through a similar sensor system 320 that can be disposed on the planar structure 211 as described before. The planar structure 211 is included in the housing 101 of the treatment device 100 as shown in Figure 1 instead of the planar structure 101 and the sensor system 120. The sensor system 320 and the treatment light source 210 (not shown in the Figures 4 and 5) are disposed in front of the device treatment window 102 in the housing 101 and in front of the skin surface 1.

The sensor system 320 comprises as sensing light source 321 two light emitting elements 322 arranged in one line with the light sensor 323. The optical arrangement 340 associated with the light sensor 332 is disposed within a tubular blind 343 of the blind 342, the tubular blind 343 surrounding the light sensor 323 and the further components of the optical arrangement 340 associated with the light sensor 323. In the direction from the light sensor 323 towards the device treatment window 102 (in Figure 5 downwards), the optical arrangement comprises further a first pinhole or slit blind 344, the lens 341, a second pinhole or slit blind 345, an absorption filter 346, a third pinhole or slit blind 347 and an interference filter 348. It is to be understood, that this buildup of the optical arrangement 340 is given as an useful example only, and that certain elements of the optical arrangement 340 might be omitted or added and/or arranged in a different order without leaving the idea of the proposal.

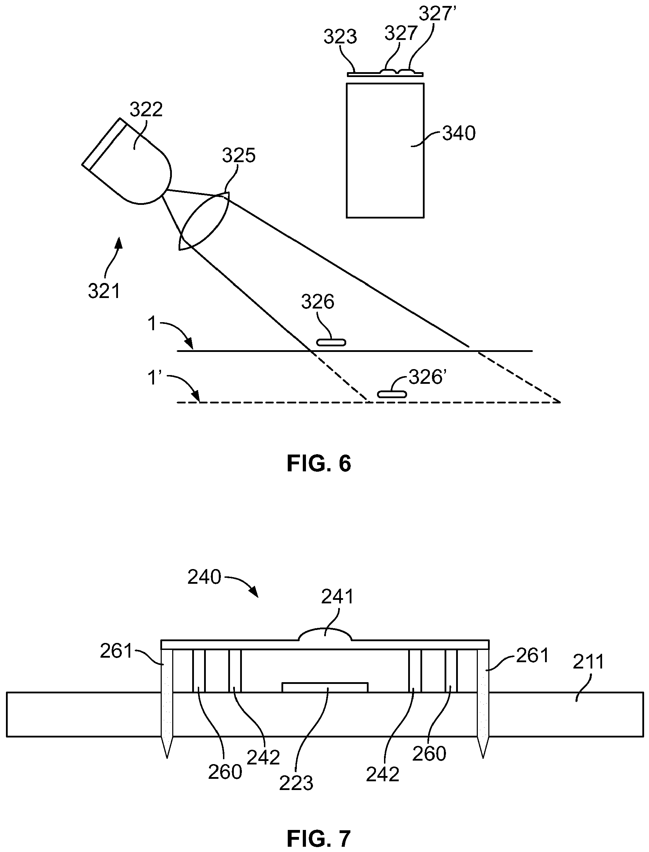

Further, the sensing light source 321 comprises at least one optical element 325 for projecting a light pattern (shown more in detail in Figure 6) on a skin surface 1 disposed outside the housing 101 in front of the device treatment window 102.

In Figure 6, the light emitting element 322 of the sensing light source 321 together with the optical element 325 for projection the light pattern 326, 326' on the skin surface 1, 1'. The light sensor 323 being in line with the invention an image light sensor (e.g. of a CCD type) reproduces the picture 327, 327' of the light pattern 326, 326'. In front of the light sensor 323, the optical arrangement 340 associated with the light sensor 323 is indicated.

The skin surface 1, 1' is shown in Figure 6 in two different distances with respect to the treatment device 100, and the light sensor 323, respectively. Dependent on the distance of the skin surface 1, 1', the projected light pattern 326, 326' appears on different surface positions and is reproduced at different picture positions 327, 327' in the image of the light sensor. Thus, it is possible by way of triangulation, for example, the determine the distance between the skin surface and the device.

The optical arrangement 140 (only described as a black box feature) might show a structure identical or similar to that described before in the examples for the optical structures 240, 340.

Figure 7 shows an example of the optical arrangement 240 as shown in Fig. 4 and its attachment to the planar structure 211. The optical arrangement 240 may be an injection molded plastic part including all or part of the optical components described before. It is positioned such that the lens 241 is positioned above the light sensor 223 (and the light emitting elements 222 not visible in Figure 7) of the sensing light source 221. The light sensor 223 is surrounded by a blind 242. The injection molded plastic part further comprises distance holders 260 to adjust the distance between the lens 241 and the light sensor 223. Further, position pins 261 are provide in engagement with the planar substrate 211 to align the optical arrangement 240 with the light sensor 223 and the light emitting elements 222 of the sensing light source 221.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A skin or hair treatment device for emitting intense light radiation (113) with an integrated sensor comprising
- a housing (101);
- a treatment light source (110, 210) disposed inside the housing (101) of the treatment device (100) for illuminating through a device treatment window (102) a surface (1), in particular a skin surface, disposed outside the housing (101) in front of the device treatment window (102);
- a sensor system (120, 220, 320) disposed inside the housing (101) of the treatment device (100), said sensor system (120, 220, 320) comprising:
• a sensing light source (121, 221, 321) for illuminating the surface (1) disposed outside the housing (101) in front of the device treatment window (102) with sensing light;
• a light sensor (123, 223, 323) for detecting the sensing light on said illuminated surface (1), the light sensor (123, 223, 323) being directed towards the illuminated surface (1) in front of the device treatment window (102) and
- a control circuit having a processor, the control circuit being adapted to control
• the treatment light source (110, 210),
• the sensing light source (121, 221, 321),
• and the light sensor (123, 223, 323)
wherein controlling of the sensing light source (121, 221, 312) comprises switching off and on a sensing light, wherein controlling of the treatment light source (110) comprises full intensity control of the high intense treatment light (113), and wherein controlling the light sensor (123, 223, 323) comprises collecting data from the light sensor (123, 223, 323) and evaluating the data, **characterized in that** the light sensor (123, 223, 323) is an image sensor and the sensor system (120, 220, 320) and the control circuit are adapted to be used for at least two, preferably all three of the following measurements of
• the skin color;
• the distance between the treatment device (100) and the surface (1);
• a movement or velocity of the treatment device (100) relative to the surface (1).

2. The skin or hair treatment device according to claim 1, **characterized in that** the treatment light source (110, 210) comprises an array of a plurality of light emitting elements (112, 222) arranged in a coplanar arrangement on a planar substrate (111, 211).

3. The skin or hair treatment device according to claim 2, **characterized in that** the light sensor (223, 323) and/or the sensing light source (221, 321) are disposed on said same planar substrate (211) as the treatment light source (210).

4. The skin or hair treatment device according to claim 2, **characterized in that** the light sensor (123) and/or the sensing light source (121) are disposed in a breakthrough (114) of the planar substrate (111) of the treatment light source (110).

5. The skin or hair treatment device according any one of the preceding claims, **characterized in that** the sensing light source (221, 321) of the sensor system (220, 320) may comprise one light emitting element (222m 322) or more light emitting elements (222, 322).

6. The skin or hair treatment device according any one of the claims 2 to 5, **characterized in that** the light sensor (223, 323) is surrounded by light emitting elements (222, 322) of the treatment light source (221, 321).

7. The skin or hair device according any one of the preceding claims, **characterized in that** the sensor system (120, 220, 320) comprises an optical arrangement (140, 240, 340) disposed in front of the light sensor (123, 223, 323).

8. The skin or hair treatment device according to claim 7, **characterized in that** the optical arrangement (140, 240, 340) comprises at least one of the following elements:
• interference filter (348);
• absorption filter (346);
• blind (242, 342, 343, 344, 345, 347);
• lens (241, 341).

9. The skin or hair treatment device according any one of the preceding claims, **characterized in that**
- the sensing light source (321) comprises at least one optical element (325) for projecting a light pattern (326) as a distinctive feature through an opening in the device housing (101) for displaying the light pattern (326) on the surface (1) disposed outside the housing (101) in front of the device treatment window (102), and
- control circuit is adapted to determine the distance between the treatment device (100) and the surface (1, 1') by determining the position and/or movement of the projected light pattern (326, 326') in the image (327, 237') of the surface (1, 1').

10. The skin or hair treatment device according any one of the preceding claims, **characterized in that**
- the sensing light source (321) comprises at least one optical element for projecting a light spot (326) through an opening in the device housing (101) for displaying the light spot (236) on the surface (1), and
- the control circuit of the treatment device (100) is adapted to select an image area of the surface (1) and to determine a signal related to the luminance value of the image (327) indicative of the melanin content of skin.
